# EUROPÄISCHE PATENTSCHRIFT

(11) **EP 4 151 178 B1**
(45) Veröffentlichungstag und Bekanntmachung des Hinweises auf die Patenterteilung: **03.07.2024**
(21) Anmeldenummer: 22196614.6
(22) Anmeldetag: 20.09.2022
(51) Int. Cl.: A61C 19/045

(54) **ANORDNUNG ZUR ERFASSUNG EINER POSITION EINES MENSCHLICHEN OBERKIEFERS UND/ODER KOPFS**
ARRANGEMENT FOR DETECTING A POSITION OF A HUMAN UPPER JAW AND/OR HEAD
DISPOSITIF DE DÉTECTION D'UNE POSITION D'UN MAXILLAIRE ET/OU D'UNE TÊTE HUMAINE

(30) Priorität: 21.09.2021 AT 507432021
(43) Veröffentlichungstag der Anmeldung: 22.03.2023
(73) Patentinhaber: STEGER, Heinrich, 39031 Bruneck (IT)
(72) Erfinder: STEGER, Heinrich, 39031 Bruneck (IT)
(74) Vertreter: Torggler & Hofmann Patentanwälte - Innsbruck

(56) Entgegenhaltungen:
- WO-A1-2020/183115
- DE-A1-102014 102 111
- DE-A1-102019 119 080
- ES-U- 1 271 360
- PL-B1- 236 744
- US-A1- 2019 298 502

## Beschreibung

Die Erfindung betrifft eine Anordnung zur Erfassung einer Position, insbesondere Roll-, Nick-, und/oder Gier-Winkel, eines menschlichen Oberkiefers und/oder Kopfs, umfassend zumindest einen ersten, an einem Oberkiefer anzuordnenden Sensor. Weiters betrifft die Erfindung ein Verfahren zur Erfassung einer Position, insbesondere Roll-, Nick-, und/oder Gier-Winkel, eines menschlichen Oberkiefers und/oder Kopfs mit einer solchen Anordnung.

Bezüglich eines menschlichen Oberkiefers und/oder Kopfs entspricht der Roll-Winkel einem Winkel um eine Sagittalachse, der Nick-Winkel einem Winkel um eine Transversalachse und der Gier-Winkel um eine Longitudinalachse.

Zur Anfertigung eines Zahnersatzes oder von Teil- bzw. Totalprothesen, insbesondere eines ästhetischen Zahnersatzes, ist es notwendig, Bewegungen von Ober- und Unterkiefer zueinander zu simulieren. Dazu werden Modelle von Ober- bzw. Unterkiefer in eine spezielle Vorrichtung zur Simulation solcher Bewegungen, einem sogenannten Artikulator, derart eingespannt, dass die Modelle von Ober- und Unterkiefer zueinander die gleiche Lage wie das tatsächliche Ober- und Unterkiefer aufweisen. Die Modelle von Ober- und Unterkiefer sind also lagerrichtig angeordnet. Der Artikulator kann ein herkömmlicher mechanischer Artikulator und die Modelle von Ober- bzw. Unterkiefer beispielsweise Gipsmodelle sein. Der Artikulator und die Modelle von Ober- bzw. Unterkiefer können aber auch digital sein.

Insbesondere für ästhetischen Zahnersatz ist es auch von Bedeutung, die Position des Kopfs zu kennen.

Die Schwierigkeit bei einem solchen Verfahren besteht darin, die Position von Ober- und Unterkiefer, insbesondere jedoch des Oberkiefers und/oder des Kopfs, zu erfassen. Im Stand der Technik wird dazu ein sogenannter Gesichtsbogen eingesetzt.

Nachteilig dabei ist einerseits, dass der Gesichtsbogen und insbesondere die mit dem Gesichtsbogen erfasste Position nur äußerst kompliziert oder nicht digital erfasst bzw. digital umgesetzt werden kann. Andererseits versperrt ein Gesichtsbogen die Sicht auf große Teile des Kopfs und der Zähne, wodurch es unmöglich wird, im Zuge der Erfassung der Position eines Oberkiefers die Zähne und den Kopf zu fotografieren. Insbesondere bei der Anfertigung eines ästhetischen Zahnersatzes ist eine solche Fotografie aber durchaus von großem Vorteil.

Aus der US 2019/0298502 A1 ist eine Vorrichtung und ein Verfahren zur Positionsbestimmung von Zähnen bekannt, wobei diese Vorrichtung zwei Sensoren aufweist, welche kabelgebunden oder kabellos mit einer Steuervorrichtung in Verbindung stehen.

Die PL 236744 B1 zeigt eine Vorrichtung zum Erfassen einer räumlichen Bewegung eines Ober- und Unterkiefers. Dabei ist eine Bissgabel vorgesehen, welche zwei Sensoren aufweist.

Die ES 1 271 360 U betrifft eine Vorrichtung zum gleichzeitigen Messen von Kiefer- und Kopfbewegungen. Ein Kinnteil E weist dabei zwei seitlich abstehende optoelektronische Plattformen auf.

Die WO 2020/183115 A1 betrifft eine Methode zur Erfassung virtueller Modelle von Ober- und Unterkieferbogen mit einem digitalen Modell eines Gesichts. Dazu wird ein Marker oder Sensor über eine Vorrichtung am Unterkiefer befestigt. Zusätzlich dazu kann am Kopf ein weiterer Marker angeordnet sein.

Die DE 10 2014 102 111 betrifft ein Verfahren zur Visualisierung zahnmedizinisch relevanter anatomischer Relationen und/oder Strukturen. Gemäß einem Ausführungsbeispiel kann ein Inertialsensorsystem vorgesehen sein, welches einem Oberkiefer starr zugeordnet ist. Dadurch können Bewegungen des Kopfes sensorisch erfasst werden. Diese Sensorsignale werden dann bei der Gewinnung der Gesamt-Bilddatensätze der Einzelbilder als Korrekturgröße berücksichtigt.

Die DE 10 2019 119 080 A1 betrifft eine Zahnschiene und ein Verfahren zur Herstellung einer solchen Zahnschiene. Dabei kann ein Messsystem zur Positions- und/oder Bewegungserfassung mit einem Bügel vorgesehen sein, welcher an einem Unterkiefer angeordnet werden kann.

Auch diese Vorrichtungen gemäß dem Stand der Technik weisen einige Nachteile auf. Unter anderem können auch bei diesen Vorrichtungen Teile des Kopfs und der Zähne verdeckt werden.

Die Aufgabe der vorliegenden Erfindung besteht darin, die beschriebenen Nachteile gemäß dem Stand der Technik zu vermeiden und eine gegenüber dem Stand der Technik verbesserte Anordnung zur Erfassung einer Position, insbesondere Roll-, Nick-, und/oder Gier-Winkel, eines menschlichen Oberkiefers und/oder Kopfs sowie ein verbessertes Verfahren zur Erfassung einer Position, insbesondere Roll-, Nick-, und/oder Gier-Winkel, eines menschlichen Oberkiefers und/oder Kopfs anzugeben.

Diese Aufgaben werden gelöst durch die Merkmale der unabhängigen Ansprüche 1, 11 und 12. Erfindungsgemäß ist es demnach vorgesehen, dass der zumindest eine erste Sensor eine Datenschnittstelle zum Übertragen und/oder Empfangen von Daten aufweist.

Durch den an einem Oberkiefer anzuordnenden ersten Sensor kann die Position des Oberkiefers erfasst werden. Die erfasste Position des Oberkiefers liegt nach Erfassung durch den Sensor digital vor und kann über die Datenschnittstelle übertragen werden. Somit steht die erfasste Position des Oberkiefers digital zur Verfügung und kann entsprechend digital weiterverarbeitet werden.

Zudem ist vorgesehen, dass die Anordnung zumindest einen zweiten, an einem Kopf anzuordnenden Sensor aufweist, wobei der zumindest eine zweite Sensor eine Datenschnittstelle zum Übertragen und/oder Empfangen von Daten aufweist.

Weiters ist vorgesehen, dass der erste Sensor und der zweite Sensor drahtlos in Datenverbindung miteinander stehen, oder dass der erste Sensor und der zweite Sensor kabelgebunden in Datenverbindung miteinander stehen.

Eine kabelgebundene Datenverbindung hat den Vorteil, dass sie äußerst kostengünstig zu realisieren ist.

Das erlaubt es, Daten zwischen dem ersten Sensor und dem zweiten Sensor auszutauschen. Insbesondere können dabei vom ersten Sensor gemessene Positionsdaten des Oberkiefers an den zweiten Sensor übertragen werden.

Da nun die Position des ersten Sensors relativ zum zweiten Sensor, als auch die Position des zweiten Sensors bekannt sind, kann der erste Sensor in weiterer Folge entfernt werden. Da Oberkiefer und Kopf eine im Wesentlichen starre Einheit bilden, bleibt die relative Position vom ersten zum zweiten Sensor gleich, unabhängig von der absoluten Position des Kopfes in einem Raum. Über die vom zweiten Sensor bekannten Positionsdaten kann also auf die Position des ersten Sensors, also des Oberkiefers, geschlossen werden.

Dadurch ist es möglich, insbesondere Fotografien oder aber auch Scans etc. des Kopfs und/oder der Zähne anzufertigen. Zudem wird eine Person, bei welcher die Position deren Oberkiefer und/oder Kopf erfasst werden soll, nicht durch den ersten, am Oberkiefer anzuordnenden Sensor eingeschränkt.

Ein erfindungsgemäßes Verfahren umfasst folgende Schritte:
- Anordnen eines ersten Sensors an einem Oberkiefer oder Kopf,
- Erfassen einer Position des Oberkiefers und/oder Kopfs über den ersten Sensor,
- Übertragen der erfassten Position des Oberkiefers und/oder Kopfs über die Datenschnittstelle.

Alternativ oder ergänzend dazu sind auch die folgenden Verfahrensschritte vorgesehen:
- Anordnen eines ersten Sensors an einem Oberkiefer,
- Anordnen eines zweiten Sensors an einem Kopf,
- Synchronisation des zweiten Sensors mit dem ersten Sensor,
- Entfernen des ersten Sensors, und
- Erfassen einer Position des Oberkiefers und/oder Kopfs über den zweiten Sensor.

Weitere vorteilhafte Ausführungsformen der Erfindung werden in den abhängigen Ansprüchen definiert.

Insbesondere kann vorgesehen sein, dass der erste Sensor und/oder der zweite Sensor ein Neigungssensor, vorzugsweise ein Beschleunigungssensor, ist. Dadurch können Roll-, Nick-, und/oder Gier-Winkel eines menschlichen Oberkiefers und/oder eines Kopfs erfasst werden.

Es kann auch vorgesehen sein, dass der erste Sensor und der zweite Sensor über Bluetooth, WLAN oder ZigBee in Datenverbindung miteinander stehen.

Das stellte eine einfache Möglichkeit zur Realisierung einer drahtlosen Verbindung dar.

In einem Ausführungsbeispiel der Erfindung kann vorgesehen sein, dass der erste Sensor und/oder der zweite Sensor einen Indikator aufweist. Der Indikator erleichtert das Ausrichten der Sensoren. Er kann vorzugsweise in Richtung des Oberkiefers zeigen. Dadurch ist eine falsche Ausrichtung der Sensoren einfach zu erkennen.

Alternativ oder ergänzend dazu kann auch vorgesehen sein, dass der erste Sensor und/oder der zweite Sensor einen Kompass aufweist. Der Kompass kann dabei auch zur Kontrolle der richtigen Ausrichtung der Sensoren herangezogen werden.

Es kann vorgesehen sein, dass der erste Sensor und/oder der zweite Sensor ein Befestigungsmittel aufweist, über welches der erste Sensor und/oder der zweite Sensor mittelbar oder unmittelbar an einem Oberkiefer und/oder einem Kopf anordenbar ist. Das Befestigungsmittel kann dabei beispielsweise in Form eines Magneten oder einer Schnapp-, Schraub-, oder Klettverbindung ausgebildet sein.

Gemäß einem Ausführungsbeispiel kann vorgesehen sein, dass die Anordnung eine erste Positioniereinrichtung aufweist, über welche der erste Sensor an einem Oberkiefer anordenbar ist.

Wenn die erste Positioniereinrichtung im Wesentlich die Form eines Konfektionslöffels aufweist, ist es möglich, den Sensor einfach und für eine Person, an deren Oberkiefer der Sensor angeordnet werden soll, angenehm an einem Oberkiefer anzuordnen.

Dazu kann die erste Positioniereinrichtung in dem Bereich, welcher an einem Oberkiefer angeordnet wird, ein Abformmaterial aufweisen. Durch das Abformmaterial wird nicht nur eine sichere Anordnung des ersten Positionierelements an einem Oberkiefer sichergestellt, sondern auch gleichzeitig ein Abdruck eines Oberkiefers bzw. von Zähnen des Oberkiefers erstellt werden.

In einer Ausführungsform der Erfindung kann die Anordnung ein an der ersten Positioniereinrichtung lösbar anordenbares Referenzelement umfassen. Das Referenzelement dient zur leichteren Erkennbarkeit der Positioniereinrichtung beim Scannen oder dergleichen der Positioniereinrichtung. Das gescannte Referenzelement kann dabei auch mit einem digitalen Modell eines Oberkiefers und/oder Kopfs in Verbindung gebracht werden, sodass das digitale Modell eines Oberkiefers und/oder Kopfs ein digitales Referenzelement aufweist.

Es könnte aber auch die erste Positioniereinrichtung allein, also ohne Referenzelement, beispielsweise gescannt werden. Das Scannen der ersten Positioniereinrichtung kann beispielsweise über einen Tischscanner oder über einen über einen Intraoralscanner erfolgen. Bei der Verwendung eines Intraoralscanners wird die Positioniereinrichtung gescannt, während sie noch an einem Oberkiefer angeordnet ist.

Beim Scannen der erste Positioniereinrichtung wird auch ein Abdruck eines Oberkiefers bzw. von Zähnen des Oberkiefers gescannt, wodurch dieser digital zur Verfügung steht.

Insbesondere kann dieses digitale Referenzelement auch an einem lagerichtig hinterlegten Referenzelement ausgerichtet werden. Das digitale Modell eines Oberkiefers und/oder Kopfs ist somit ebenfalls lagerichtig positioniert und ausgerichtet.

Wenn das Referenzelement eine unregelmäßige Oberfläche aufweist, ist es leichter zu erfassen, beispielsweise über einen Scan.

Weiters kann auch vorgesehen sein, dass die Anordnung eine zweite Positioniereinrichtung aufweist, über welche der zweite Sensor an einem Kopf anordenbar ist.

Insbesondere kann die zweite Positioniereinrichtung bügelförmig oder nach Art eines Stirnbandes ausgebildet sein. Das stellt eine einfache und kostengünstige Möglichkeit zur Realisierung einer zweiten Positioniereinrichtung dar.

Bevorzugt kann vorgesehen sein, dass die Anordnung eine Kameraeinrichtung aufweist und besonders bevorzugt, dass die Kameraeinrichtung einen Neigungssensor, vorzugsweise einen Beschleunigungssensor, aufweist.

Über eine Kameraeinrichtung können Fotografien eines Kopfs oder von Zähnen angefertigt werden, welche in Verbindung mit der erfassten Position eines Oberkiefers und/oder Kopfs zur Anfertigung eines ästhetischen Zahnersatzes herangezogen werden können. Insbesondere können frontale uns seitliche Fotografien angefertigt werden.

Über den Neigungssensor der Kameraeinrichtung kann die Position der Kamera bei der Aufnahme der Fotografie und demnach auch die Position des Bildes relativ zur erfassten Position eines Oberkiefers und/oder Kopfs bestimmt werden.

Die Fotografie und ein Modell eines Oberkiefers und/oder Kopfs können demnach lagerichtig zueinander positioniert werden, was das Anfertigen eines ästhetischen Zahnersatzes erleichtert.

Vorteilhafterweise kann vorgesehen sein, dass die Kameraeinrichtung bevorzugt drahtlos, besonders bevorzugt über Bluetooth, WLAN oder ZigBee, in Datenverbindung mit dem ersten Sensor und/oder dem zweiten Sensor steht. Über eine solche Datenverbindung können (Positions-)Daten der Sensoren bzw. der Kamera zwischen Sensoren und der Kamera ausgetauscht werden. Alternativ könnte die Datenverbindung auch kabelgebunden realisiert werden.

Besonders bevorzugt kann vorgesehen sein, dass die Kameraeinrichtung ein Smartphone ist. Das bringt einerseits den Vorteil mit sich, dass bereits ein Neigungssensor und Möglichkeiten zum Herstellen einer Datenverbindung im Smartphone vorhanden sind. Eine aufwendige und kostenintensive Nachrüstung bestehender Kamerasystem entfällt.

Andererseits sind Smartphones weit verbreitet, weshalb die zusätzliche Anschaffung einer Kameraeinrichtung zu den Sensoren entfällt. Ein Nutzer kann einfach sein eigenes Smartphone als Kameraeinrichtung verwenden.

Besonders bevorzugt kann auch vorgesehen sein, dass die Anordnung eine Datenverarbeitungseinrichtung aufweist. Der Datenverarbeitungseinrichtung kommt dabei eine Vielzahl an Aufgaben zu:
Die Datenverarbeitungseinrichtung kann zur Verarbeitung von Sensordaten des ersten Sensors und/oder des zweiten Sensors ausgebildet sein.

Dazu kann vorgesehen sein, dass die Datenverarbeitungsvorrichtung bevorzugt drahtlos, besonders bevorzugt über Bluetooth, WLAN oder ZigBee, in Datenverbindung mit dem ersten Sensor und/oder dem zweiten Sensor steht.

Unter die Verarbeitung von Sensordaten kann beispielsweise die lagerichtige Positionierung von digitalen Modellen eines Ober- und Unterkiefers bzw. eines Kopfs mittels der Sensordaten fallen.

Die Datenverarbeitungseinrichtung kann weiters zur Verarbeitung von Daten der Kameraeinrichtung ausgebildet sein.

Dazu kann vorgesehen sein, dass die Datenverarbeitungsvorrichtung bevorzugt drahtlos, besonders bevorzugt über Bluetooth, WLAN oder ZigBee, in Datenverbindung mit der Kameraeinrichtung steht.

Unter die Verarbeitung von der Kameraeinrichtung können beispielsweise die lagerichtige Positionierung von Fotografien relativ zu digitalen Modellen eines Ober- und Unterkiefers bzw. eines Kopfs und/oder die Skalierung besagter Fotografien an digitale Modelle eines Ober- und Unterkiefers bzw. eines Kopfs fallen.

Die Datenverarbeitungseinrichtung kann zur weiteren Verarbeitung dieser Daten eingesetzt werden. Beispielsweise kann die Datenverarbeitungseinrichtung einen digitalen Artikulator umfassen, in welchem mittels Modellen von Ober- und Unterkiefer Bewegungen der von Ober- und Unterkiefer relativ zueinander digital simuliert werden können.

Alternativ oder ergänzend dazu kann die Datenverarbeitungseinrichtung auch zur Ausgabe von in einem mechanischen Artikulator verwertbaren Daten ausgebildet sein. Mithilfe dieser Daten können beispielsweise (Gips-)Modelle von Ober- und Unterkiefer lagerichtig in einem mechanischen Artikulator angeordnet und somit Bewegungen von Ober- und Unterkiefer im mechanischen Artikulator simuliert werden.

Hinsichtlich eines erfindungsgemäßen Verfahrens kann vorgesehen sein, dass der erste Sensor vor dem ersten Verfahrensschritt kalibriert wird. Somit kann sichergestellt werden, dass der erste Sensor vor dem ersten Verfahrensschritt eine richtige Nullposition aufweist.

Bevorzugt kann vorgesehen sein, dass als weiterer Schritt über eine Kameraeinrichtung zumindest eine Fotografie, vorzugsweise zumindest jeweils eine frontale und eine seitliche Fotografie, des Oberkiefers und/oder Kopfs aufgenommen wird.

Besonders bevorzugt kann vorgesehen sein, dass bei der Aufnahme der zumindest einen Fotografie eine Position, insbesondere Roll-, Nick-, und/oder Gier-Winkel, der Kameraeinrichtung über einen Neigungssensor, vorzugsweise einem Beschleunigungssensor, der Kameraeinrichtung erfasst wird.

Dadurch ist die Position der Kameraeinrichtung und demnach auch der Fotografie zum Zeitpunkt ihrer Aufnahme bekannt. Nachdem auch die Position eines Oberkiefers und/oder Kopfs bekannt ist, können die Fotografie und ein Modell eines Oberkiefers und/oder Kopfs lagerichtig zueinander ausgerichtet werden.

Dazu kann vorgesehen sein, dass die zumindest eine Fotografie und/oder eine Position der zumindest einen Fotografie von der Kameraeinrichtung auf eine Datenverarbeitungseinrichtung übertragen wird.

Es kann auch vorgesehen sein, dass in der Datenverarbeitungseinrichtung ein Modell eines Oberkiefers und/oder Kopfs über die erfasste Position des Oberkiefers und/oder Kopfs lagerichtig positioniert wird. Dies kann manuell durch einen Benutzer oder automatisch durch die Datenverarbeitungseinrichtung erfolgen.

Dazu kann vorgesehen sein, dass die erfasste Position des Oberkiefers und/oder Kopfs von dem ersten Sensor oder dem zweiten Sensor auf eine Datenverarbeitungseinrichtung übertragen wird.

Es kann weiters vorgesehen sein, dass die zumindest eine Fotografie in Abhängigkeit des Modells des Oberkiefers und/oder Kopfs skaliert wird. Hierzu können beispielsweise Markierungen, sogenannte Marker, deren Abstände zueinander bekannt sind, an einem Kopf angeordnet werden. Über diese Marker und den bekannten Abstand kann eine Fotografie skaliert werden.

Somit ist es einerseits möglich, ein Modell eine des Oberkiefers und/oder Kopfs sowie ein entsprechendes Modell eines Unterkiefers lagerichtig zu positionieren.

Andererseits kann dabei auch eine Fotografie, oder auch mehrere Fotografien, beispielsweise frontal und von beiden Seiten, lagerichtig und in richtiger Größe dem Modell bzw. den Modellen zugeordnet sein.

Somit hat ein Anwender optische (Fotografie) sowie strukturelle (Modelle) Merkmale zusammenhängend zur Verfügung, was die Anfertigung eines, insbesondere ästhetischen, Zahnersatzes signifikant vereinfacht.

Weitere Einzelheiten und Vorteile der Erfindung werden anhand der Figurenbeschreibung unter Bezugnahme auf die Zeichnungen im Folgenden näher erläutert. Darin zeigen:
- Fig. 1a: schematische Draufsicht eines Sensors,
- Fig. 1b: schematische Vorderansicht eines Sensors,
- Fig. 1c: schematische Untersicht eines Sensors,
- Fig. 2a: schematische Draufsicht eine ersten Positioniereinrichtung mit einem ersten Sensor,
- Fig. 2b: schematische Draufsicht einer ersten Positioniereinrichtung mit einem Referenzelement,
- Fig. 2c: schematische Draufsicht einer ersten Positioniereinrichtung,
- Fig. 3a: schematische Draufsicht einer zweiten Positioniereinrichtung mit einem zweiten Sensor,
- Fig. 3b: schematische Vorderansicht einer ersten Positioniereinrichtung mit einem zweiten Sensor,
- Fig. 4a: eine schematische Vorderansicht einer Anordnung,
- Fig. 4b: eine schematische Seitenansicht einer Anordnung,
- Fig. 5a: eine schematische Seitenansicht einer Anordnung bei einem Schritt eines Verfahrens,
- Fig. 5b: eine schematische Seitenansicht einer Anordnung bei einem weiteren Schritt eines Verfahrens,
- Fig. 5c: eine schematische Seitenansicht einer Anordnung bei einem weiteren Schritt eines Verfahrens, und
- Fig. 5d: eine schematische Seitenansicht einer Anordnung bei einem weiteren Schritt eines Verfahrens.

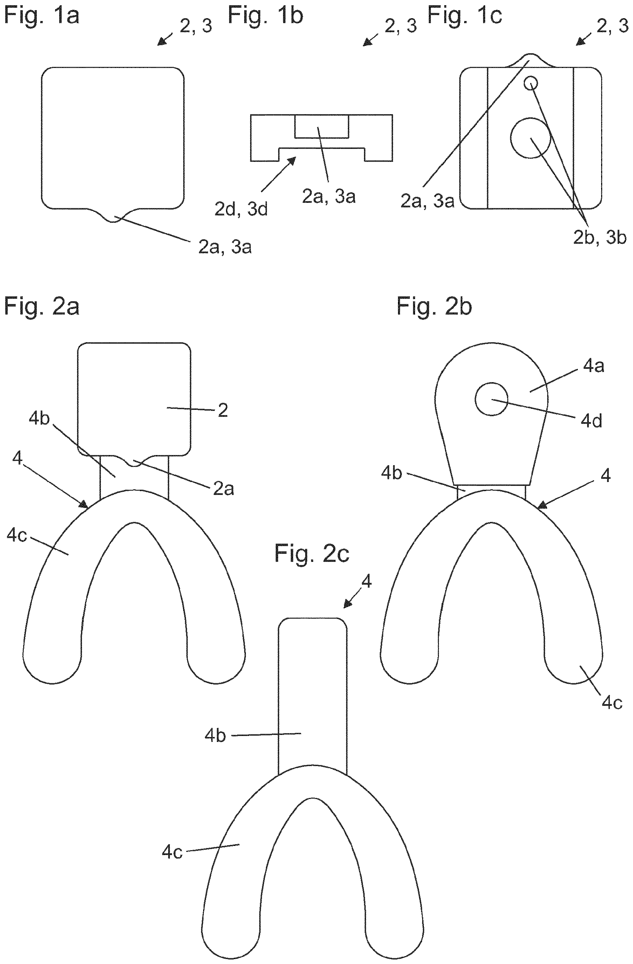

Die Figur 1a zeigt eine schematische Draufsicht eines Sensors 2, 3, die Figur 1b die entsprechende Vorderansicht und die Figur 1c die entsprechende Untersicht.

Im vorliegenden Ausführungsbeispiel ist ein Sensor 2, 3 im wesentlichen quaderförmig ausgebildet. Die Form des Sensors 2, 3 ist aber im Wesentlichen nicht von Relevanz und kann beliebig ausgebildet sein.

Der Sensor 2, 3 weist einen Indikator 2a, 3a in Form eines Vorsprungs auf. Der Sensor 2, 3 wird so positioniert, dass der Indikator 2a, 3a in Richtung eines Oberkiefers weist. Der Indikator 2a, 3a kann beispielsweise auch durch einen Kompass ersetzt oder ergänzt werden.

An der Unterseite des Sensor 2, 3 ist eine Ausnehmung 2d, 3d zu erkennen, in welcher ein Befestigungsmittel 2b, 3b angeordnet ist. Im vorliegenden Beispiel wird das Befestigungsmittel 2b, 3b einerseits durch einen Magneten in der Mitte der Ausnehmung 2d, 3d und andererseits durch eine Rastnoppe am Rand der Ausnehmung 2d, 3d gebildet.

Das Befestigungsmittel 2b, 3b kann natürlich nur einen Magneten oder nur eine Rastnoppe oder auch jede andere geeignete Verbindungsvorrichtung aufweisen. Beispielsweise genannt seien eine Schraub- oder Schnappverbindung.

Die Ausnehmung 2d, 3d dient zur Aufnahme einer Zunge 4b einer ersten Positioniereinrichtung 4 oder eines Verbindungselements 5a einer zweiten Positioniereinrichtung 5.

Durch die Ausnehmung 2d, 3d reine eine Verdrehung des Sensors 2, 3 relativ zur Zunge 4b oder zum Verbindungselement 5a verhindert werden. Die Ausnehmung stellt also eine Arretierung des Sensors 2, 3 dar.

Die Figur 2a zeigt schematische Draufsicht einer ersten Positioniereinrichtung 4 mit einem ersten Sensor 2, die Figur 2b eine Darstellung mit einem erfindungsmäßen Referenzelement 4a, und die Figur 3c nur eine entsprechende erste Positioniereinrichtung 4.

Es ist erkennbar, dass die erste Positioniereinrichtung 4 im Wesentlichen die Form eines Konfektionslöffels aufweist. Die erste Positioniereinrichtung 4 weist dabei ein Mundstück 4c auf, auf welches eine Person, von welcher die Position ihres Oberkiefers und/oder Kopfs erfasst werden soll, beißen kann.

Dadurch wird der erste Sensor 2 über die Positioniereinrichtung 4 am Oberkiefer der Person angeordnet. Die Zunge 4b dient zur Anordnung des ersten Sensor 2 an der ersten Positioniereinrichtung 4.

Es ist auch ersichtlich, wie ein Referenzelement 4a an der Zunge 4b der ersten Positioniereinrichtung 4 angeordnet werden kann. Das Referenzelement 4a weist dabei ein Befestigungsmittel 4d in Form eines Magneten auf.

Die Zunge 4b ist im vorliegenden Fall aus einem magnetischen oder magnetisierbaren Material ausgebildet. Grundsätzlich ist in Abhängigkeit der Befestigungsmittel 2b, 3b bzw. 4d aber jedes geeignete Material denkbar.

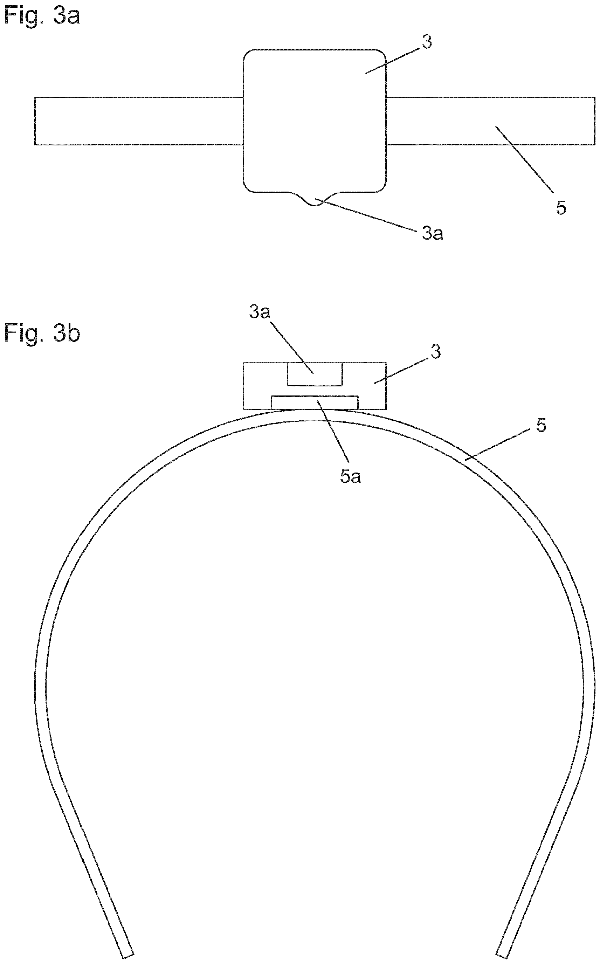

Die Figur 3a zeigt eine schematische Draufsicht einer zweiten Positioniereinrichtung 5 mit einem zweiten Sensor 3 und die Figur 3b eine entsprechende Vorderansicht dazu.

Es ist erkennbar, dass die zweite Positioniereinrichtung 5 im vorliegenden Ausführungsbeispiel bügelförmig ausgebildet ist. Dabei ist das Material der Positioniereinrichtung 5 ausreichend elastisch verformbar, um sich an einen Kopf anzupassen.

Beispielsweise kann die zweite Positioniereinrichtung 5 aus einem Kunststoff, insbesondere additiv, gefertigt sein.

Die zweite Positioniereinrichtung 5 kann aber beispielsweise auch nach Art eines Stirnbandes bzw. im Wesentlichen analog zu einer Bebänderung einer Stirnlampe ausgebildet sein. Grundsätzlich ist jede Form geeignet, mittels welcher der zweite Sensor 4 bewegungsgekoppelt an einem Kopf angeordnet werden kann.

Die zweite Positioniereinrichtung 5 weist ein Verbindungselement 5a zum Anordnen des zweiten Sensors 3 an der zweiten Positioniereinrichtung 5 auf. Das Verbindungselement 5a kann dabei ein zum Befestigungsmittel 3a des zweiten Sensors 3 korrespondierendes Befestigungsmittel aufweisen. Im vorliegenden Ausführungsbeispiel kann das Verbindungselement 5a beispielsweise zumindest teilweise magnetisch oder magnetisierbar ausgebildet sein.

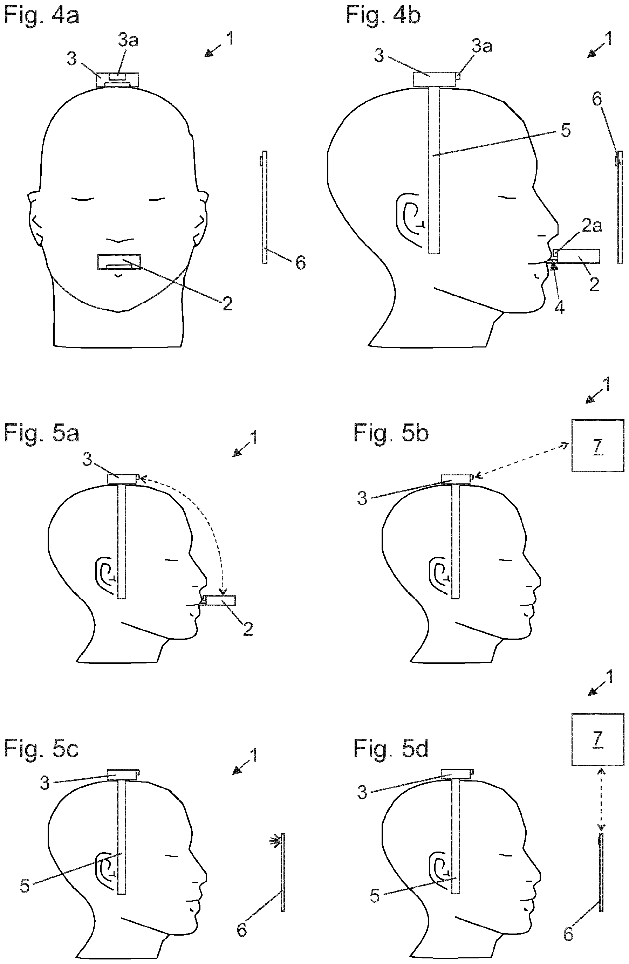

Die Figur 4a zeigt eine schematische Vorderansicht einer Anordnung 1, die Figur 4b eine entsprechende Seitenansicht dazu. Es ist erkennbar, dass ein erster Sensor 2 über eine erste Positioniereinrichtung 4 an einem Oberkiefer angeordnet ist. Ein zweiter Sensor 3 ist über eine zweite Positioniereinrichtung 5 am Kopf angeordnet.

Es ist erkennbar, dass Indikatoren 2a, 2b des ersten Sensors 2 bzw. des zweiten Sensors 3 in Richtung eines Oberkiefers zeigen. Die Sensoren 2, 3 sind demnach richtig ausgerichtet.

Es ist auch eine Kameraeinrichtung 6 in Form eines Smartphones erkennbar. Die Kameraeinrichtung 6 kann dabei frontale oder auch seitliche Fotografien aufnehmen.

Die Figur 5a zeigt eine schematische Seitenansicht einer Anordnung 1 bei einem Schritt eines Verfahrens.

Es ist erkennbar, dass der erste Sensor 2 bereits an einem Oberkiefer und der zweite Sensor 3 bereits an einem Kopf angeordnet sind. Die beiden Sensoren 2, 3 stehen in Datenverbindung miteinander (gekennzeichnet durch die strichlierte Linie), wobei der zweite Sensor 3 mit dem ersten Sensor 2 synchronisiert wird.

Der zweite Sensor 3 kennt somit die Position in Form von Roll-, Nick-, und Gier-Winkel des zweiten Sensors 2 relativ zu sich selbst sowie seine eigene Position. Ausgehend von der Position des zweiten Sensor 3 kann also auf die Position des zweiten Sensors 2, also der Position des Oberkiefers, geschlossen werden.

Die Figur 5b zeigt eine schematische Seitenansicht einer Anordnung 1 bei einem weiteren Schritt eines Verfahrens. Es ist erkennbar, dass der erste Sensor 2 nicht mehr am Oberkiefer angeordnet ist.

Nachdem über den zweiten Sensor 3 auf die Position des ersten Sensors 2 bzw. des Oberkiefers geschlossen werden kann, kann auch nur mit dem zweiten Sensor 3 die Position des Oberkiefers und über den zweiten Sensor 3 auch die Position des Kopfs erfasst werden.

Die erfassten Daten werden im vorliegenden Ausführungsbeispiel drahtlos an eine Datenverarbeitungseinrichtung 7 übertragen. Es ist aber auch eine kabelgebundene Übertragung denkbar.

Die Figur 5c zeigt eine schematische Seitenansicht einer Anordnung 1 bei einem weiteren Schritt eines Verfahrens.

Es ist die Kameraeinrichtung 6 in Form eines Smartphones erkennbar. Beim Aufnehmen einer Fotografie wird auch die Position der Kameraeinrichtung 6 über einen Neigungssensor der Kameraeinrichtung 6 erfasst.

Die Figur 5d zeigt eine schematische Seitenansicht einer Anordnung 1 bei einem weiteren Schritt eines Verfahrens.

Es ist erkennbar, dass die aufgenommenen Fotografien sowie erfassten Positionsdaten der Kameraeinrichtung 6 nach Aufnahme der Fotografien drahtlos an die Datenverarbeitungseinrichtung 7 übertragen. Es ist aber auch eine kabelgebundene Übertragung denkbar.

In der Datenverarbeitungseinrichtung 7 können die erfassten Daten, wie bereits beschrieben, weiterverarbeitet werden.

### Bezugszeichenliste:

- 1: Anordnung
- 2: Erster Sensor

- 2a: Indikator
- 2b: Befestigungsmittel
- 2c: Datenschnittstelle
- 2d: Ausnehmung

- 3: Zweiter Sensor

- 3a: Indikator
- 3b: Befestigungsmittel
- 3c: Datenschnittstelle
- 3d: Ausnehmung

- 4: Erste Positioniereinrichtung

- 4a: Referenzelement
- 4b: Zunge
- 4c: Mundstück
- 4d: Befestigungsmittel

- 5: Zweite Positioniereinrichtung

- 5a: Verbindungselement

- 6: Kameraeinrichtung
- 7: Datenverarbeitungsvorrichtung

## Patentansprüche

1. Anordnung (1) zur Erfassung einer Position, insbesondere Roll-, Nick-, und/oder Gier-Winkel, eines menschlichen Oberkiefers und/oder Kopfs, umfassend zumindest einen ersten, an einem Oberkiefer anzuordnenden Sensor (2), wobei der zumindest eine erste Sensor (2) eine Datenschnittstelle (2c) zum Übertragen und/oder Empfangen von Daten aufweist, wobei die Anordnung (1) zumindest einen zweiten, an einem Kopf anzuordnenden Sensor (3) aufweist, wobei der zumindest eine zweite Sensor eine Datenschnittstelle (3c) zum Übertragen und/oder Empfangen von Daten aufweist, **dadurch gekennzeichnet, dass** der erste Sensor (2) und der zweite Sensor (3) drahtlos in Datenverbindung miteinander stehen, oder dass der erste Sensor (2) und der zweite Sensor (3) kabelgebunden in Datenverbindung miteinander stehen.

2. Anordnung nach Anspruch 1, **dadurch gekennzeichnet, dass** der erste Sensor (2) und/oder der zweite Sensor (3)
- ein Neigungssensor, vorzugsweise ein Beschleunigungssensor, ist, und/oder
- einen Indikator (2a, 3a) aufweist, und/oder
- einen Kompass aufweist, und/oder
- der erste Sensor (2) und/oder der zweite Sensor (3) ein Befestigungsmittel (2b, 3b) aufweist, über welche der erste Sensor (2) und/oder der zweite Sensor (3) mittelbar oder unmittelbar an einem Oberkiefer und/oder einem Kopf anordenbar ist.

3. Anordnung nach einem der Ansprüche 1 oder 2, **dadurch gekennzeichnet, dass** der erste Sensor (2) und der zweite Sensor (3) über Bluetooth, WLAN oder ZigBee in Datenverbindung miteinander stehen.

4. Anordnung nach einem der Ansprüche 1 bis 3, **dadurch gekennzeichnet, dass** die Anordnung (1) eine erste Positioniereinrichtung (4) aufweist, über welche der erste Sensor (2) an einem Oberkiefer anordenbar ist, wobei die erste Positioniereinrichtung (4) vorzugsweise im Wesentlich die Form eines Konfektionslöffels aufweist.

5. Anordnung nach Anspruch 4, **dadurch gekennzeichnet, dass** die Anordnung (1) ein an der ersten Positioniereinrichtung (4) lösbar anordenbares Referenzelement (4a) umfasst, wobei das Referenzelement (4a) vorzugsweise eine unregelmäßige Oberfläche aufweist.

6. Anordnung nach einem der Ansprüche 1 bis 5, **dadurch gekennzeichnet, dass** die Anordnung (1) eine zweite Positioniereinrichtung (5) aufweist, über welche der zweite Sensor (2) an einem Kopf anordenbar ist, wobei die zweite Positioniereinrichtung (5) vorzugsweise bügelförmig oder nach Art eines Stirnbandes ausgebildet ist.

7. Anordnung nach einem der Ansprüche 1 bis 6, **dadurch gekennzeichnet, dass** die Anordnung (1) eine Kameraeinrichtung (6) aufweist, wobei die Kameraeinrichtung (6) vorzugsweise
- einen Neigungssensor, vorzugsweise einen Beschleunigungssensor, aufweist, und/oder
- bevorzugt drahtlos, besonders bevorzugt über Bluetooth, WLAN oder ZigBee, in Datenverbindung mit dem ersten Sensor (2) und/oder dem zweiten Sensor (3) steht, und/oder
- ein Smartphone ist.

8. Anordnung nach einem der Ansprüche 1 bis 7, **dadurch gekennzeichnet, dass** die Anordnung (1) eine Datenverarbeitungseinrichtung (7) aufweist, wobei die Datenverarbeitungsvorrichtung (7) vorzugsweise:
- bevorzugt drahtlos, besonders bevorzugt über Bluetooth, WLAN oder ZigBee, in Datenverbindung mit dem ersten Sensor (2) und/oder dem zweiten Sensor (3) steht, und/oder
- bevorzugt drahtlos, besonders bevorzugt über Bluetooth, WLAN oder ZigBee, in Datenverbindung mit der Kameraeinrichtung (6) steht.

9. Anordnung nach Anspruch 8, **dadurch gekennzeichnet, dass** die Datenverarbeitungseinrichtung (7) zur Verarbeitung von Sensordaten des ersten Sensors (2) und/oder des zweiten Sensors (3) und/oder zur Verarbeitung von Daten der Kameraeinrichtung (6) ausgebildet ist.

10. Anordnung nach einem der Ansprüche 8 oder 9, **dadurch gekennzeichnet, dass** die Datenverarbeitungseinrichtung (7) einen digitalen Artikulator umfasst, und/oder zur Ausgabe von in einem mechanischen Artikulator verwertbaren Daten ausgebildet ist.

11. Verfahren zur Erfassung einer Position, insbesondere Roll-, Nick-, und/oder Gier-Winkel, eines menschlichen Oberkiefers und/oder Kopfs mit einer Anordnung nach einem der Ansprüche 1 bis 10, **gekennzeichnet durch** folgende Schritte:
- Anordnen eines ersten Sensors (2) an einem Oberkiefer oder Kopf,
- Erfassen einer Position des Oberkiefers und/oder Kopfs über den ersten Sensor (2),
- Übertragen der erfassten Position des Oberkiefers und/oder Kopfs über die Datenschnittstelle (2c),
- vorzugsweise wobei der erste Sensor (2) vor dem ersten Verfahrensschritt kalibriert wird.

12. Verfahren zur Erfassung einer Position, insbesondere Roll-, Nick-, und/oder Gier-Winkel, eines menschlichen Oberkiefers und/oder Kopfs mit einer Anordnung nach einem der Ansprüche 1 bis 10, **gekennzeichnet durch** folgende Schritte:
- Anordnen eines ersten Sensors (2) an einem Oberkiefer,
- Anordnen eines zweiten Sensors (3) an einem Kopf,
- Synchronisation des zweiten Sensors (3) mit dem ersten Sensor (2),
- Entfernen des ersten Sensors (2), und
- Erfassen einer Position des Oberkiefers und/oder Kopfs über den zweiten Sensor (3),
- vorzugsweise wobei der erste Sensor (2) vor dem ersten Verfahrensschritt kalibriert wird.

13. Verfahren nach einem der Ansprüche 11 oder 12, **dadurch gekennzeichnet, dass** als weiterer Schritt über eine Kameraeinrichtung (6) zumindest eine Fotografie, vorzugsweise zumindest jeweils eine frontale und eine seitliche Fotografie, des Oberkiefers und/oder Kopfs aufgenommen wird, vorzugsweise wobei
- bei der Aufnahme der zumindest einen Fotografie eine Position, insbesondere Roll-, Nick-, und/oder Gier-Winkel, der Kameraeinrichtung (6) über einen Neigungssensor, vorzugsweise einem Beschleunigungssensor, der Kameraeinrichtung (6) erfasst wird, und/oder
- die zumindest eine Fotografie und/oder eine Position der zumindest einen Fotografie von der Kameraeinrichtung (6) auf eine Datenverarbeitungseinrichtung (7) übertragen wird.

14. Verfahren nach einem der Ansprüche 11 bis 13, **dadurch gekennzeichnet, dass** die erfasste Position des Oberkiefers und/oder Kopfs von dem ersten Sensor (2) oder dem zweiten Sensor (3) auf eine Datenverarbeitungseinrichtung (7) übertragen wird, vorzugsweise wobei in der Datenverarbeitungseinrichtung (7) ein Modell eines Oberkiefers und/oder Kopfs über die erfasste Position des Oberkiefers und/oder Kopfs lagerichtig positioniert wird.

15. Verfahren nach den Ansprüchen 13 und 14, **dadurch gekennzeichnet, dass** in der Datenverarbeitungseinrichtung (7) die zumindest eine Fotografie über die erfasste Position der Fotografie lagerichtig zu dem Modell des Oberkiefers und/oder Kopfs positioniert wird, vorzugsweise wobei die zumindest eine Fotografie in Abhängigkeit des Modells des Oberkiefers und/oder Kopfs skaliert wird.

## Claims

1. Arrangement (1) for detecting a position, in particular a roll, pitch and/or yaw angle, of a human upper jaw and/or head, comprising at least one first sensor (2) to be arranged on an upper jaw, wherein the at least one first sensor (2) has a data interface (2c) for transmitting and/or receiving data, wherein the arrangement (1) has at least one second sensor (3) to be arranged on a head, wherein the at least one second sensor has a data interface (3c) for transmitting and/or receiving data, **characterized in that** the first sensor (2) and the second sensor (3) have a wireless data connection to each other, or **in that** the first sensor (2) and the second sensor (3) have a wired data connection to each other.

2. Arrangement according to claim 1, **characterized in that** the first sensor (2) and/or the second sensor (3)
- is a tilt sensor, preferably an acceleration sensor, and/or
- has an indicator (2a, 3a), and/or
- has a compass, and/or
- the first sensor (2) and/or the second sensor (3) has a fastening means (2b, 3b), via which the first sensor (2) and/or the second sensor (3) can be arranged indirectly or directly on an upper jaw and/or a head.

3. Arrangement according to one of claims 1 or 2, **characterized in that** the first sensor (2) and the second sensor (3) have a data connection to each other via Bluetooth, WLAN or Zigbee.

4. Arrangement according to one of claims 1 to 3, **characterized in that** the arrangement (1) has a first positioning device (4), via which the first sensor (2) can be arranged on an upper jaw, wherein the first positioning device (4) preferably substantially has the shape of an impression tray.

5. Arrangement according to claim 4, **characterized in that** the arrangement (1) comprises a reference element (4a) which can be detachably arranged on the first positioning device (4), wherein the reference element (4a) preferably has an irregular surface.

6. Arrangement according to one of claims 1 to 5, **characterized in that** the arrangement (1) has a second positioning device (5), via which the second sensor (2) can be arranged on a head, wherein the second positioning device (5) is preferably formed U-shaped or in the style of a headband.

7. Arrangement according to one of claims 1 to 6, **characterized in that** the arrangement (1) has a camera device (6), wherein the camera device (6) preferably
- has a tilt sensor, preferably an acceleration sensor, and/or
- has a, preferably wireless, data connection to the first sensor (2) and/or the second sensor (3), particularly preferably via Bluetooth, WLAN or Zigbee, and/or
- is a smartphone.

8. Arrangement according to one of claims 1 to 7, **characterized in that** the arrangement (1) has a data processing device (7), wherein the data processing device (7) preferably:
- has a, preferably wireless, data connection to the first sensor (2) and/or the second sensor (3), particularly preferably via Bluetooth, WLAN or Zigbee, and/or
- has a, preferably wireless, data connection to the camera device (6), particularly preferably via Bluetooth, WLAN or Zigbee.

9. Arrangement according to claim 8, **characterized in that** the data processing device (7) is formed to process sensor data from the first sensor (2) and/or the second sensor (3) and/or to process data from the camera device (6).

10. Arrangement according to one of claims 8 or 9, **characterized in that** the data processing device (7) comprises a digital articulator, and/or is formed to output data utilizable in a mechanical articulator.

11. Method for detecting a position, in particular a roll, pitch and/or yaw angle, of a human upper jaw and/or head with an arrangement according to one of claims 1 to 10, **characterized by** the following steps:
- arranging a first sensor (2) on an upper jaw or head,
- detecting a position of the upper jaw and/or head via the first sensor (2),
- transmitting the detected position of the upper jaw and/or head via the data interface (2c),
- preferably wherein the first sensor (2) is calibrated before the first method step.

12. Method for detecting a position, in particular a roll, pitch and/or yaw angle, of a human upper jaw and/or head with an arrangement according to one of claims 1 to 10, **characterized by** the following steps:
- arranging a first sensor (2) on an upper jaw,
- arranging a second sensor (3) on a head,
- synchronizing the second sensor (3) with the first sensor (2),
- removing the first sensor (2), and
- detecting a position of the upper jaw and/or head via the second sensor (3),
- preferably wherein the first sensor (2) is calibrated before the first method step.

13. Method according to one of claims 11 or 12, **characterized in that**, as a further step, at least one photograph, preferably in each case at least one frontal and one lateral photograph, of the upper jaw and/or head is taken via a camera device (6), preferably wherein,
- during the taking of the at least one photograph, a position, in particular a roll, pitch and/or yaw angle, of the camera device (6) is detected via a tilt sensor, preferably an acceleration sensor, of the camera device (6), and/or
- the at least one photograph and/or a position of the at least one photograph is transmitted from the camera device (6) to a data processing device (7).

14. Method according to one of claims 11 to 13, **characterized in that** the detected position of the upper jaw and/or head is transmitted from the first sensor (2) or the second sensor (3) to a data processing device (7), preferably wherein, in the data processing device (7), a model of an upper jaw and/or head is positioned in the correct position via the detected position of the upper jaw and/or head.

15. Method according to claims 13 and 14, **characterized in that**, in the data processing device (7), the at least one photograph is positioned in the correct position in relation to the model of the upper jaw and/or head via the detected position of the photograph, preferably wherein the at least one photograph is scaled depending on the model of the upper jaw and/or head.

## Revendications

1. Ensemble (1) pour la détection d'une position, en particulier d'un angle de roulis, de tangage, et/ou de lacet, d'un(e) maxillaire supérieur et/ou tête humain(e), comprenant au moins un premier capteur (2) à disposer sur un maxillaire supérieur, dans lequel l'au moins un premier capteur (2) présente une interface de données (2c) pour la transmission et/ou réception de données, dans lequel l'ensemble (1) présente au moins un deuxième capteur (3) à disposer sur une tête, dans lequel l'au moins un deuxième capteur présente une interface de données (3c) pour la transmission et/ou réception de données, **caractérisé en ce que** le premier capteur (2) et le deuxième capteur (3) sont en liaison de données l'un avec l'autre sans fil, ou que le premier capteur (2) et le deuxième capteur (3) sont en liaison de données l'un avec l'autre de manière filaire.

2. Ensemble selon la revendication 1, **caractérisé en ce que** le premier capteur (2) et/ou le deuxième capteur (3)
- est un capteur d'inclinaison, de préférence un capteur d'accélération, et/ou
- présente un indicateur (2a, 3a), et/ou
- présente une boussole, et/ou
- le premier capteur (2) et/ou le deuxième capteur (3) présente un moyen de fixation (2b, 3b), par l'intermédiaire duquel le premier capteur (2) et/ou le deuxième capteur (3) peut être disposé indirectement ou directement sur un maxillaire supérieur et/ou une tête.

3. Ensemble selon l'une quelconque des revendications 1 ou 2, **caractérisé en ce que** le premier capteur (2) et le deuxième capteur (3) sont en liaison de données l'un avec l'autre par Bluetooth, WLAN ou ZigBee.

4. Ensemble selon l'une quelconque des revendications 1 à 3, **caractérisé en ce que** l'ensemble (1) présente un premier dispositif de positionnement (4), par l'intermédiaire duquel le premier capteur (2) peut être disposé sur un maxillaire supérieur, dans lequel le premier dispositif de positionnement (4) présente de préférence sensiblement la forme d'un aligneur.

5. Ensemble selon la revendication 4, **caractérisé en ce que** l'ensemble (1) comprend un élément de référence (4a) pouvant être disposé de manière détachable sur le premier dispositif de positionnement (4), dans lequel l'élément de référence (4a) présente de préférence une surface irrégulière.

6. Ensemble selon l'une quelconque des revendications 1 à 5, **caractérisé en ce que** l'ensemble (1) présente un deuxième dispositif de positionnement (5), par l'intermédiaire duquel le deuxième capteur (2) peut être disposé sur une tête, dans lequel le deuxième dispositif de positionnement (5) est réalisé de préférence en forme d'étrier ou à la façon d'un bandeau.

7. Ensemble selon l'une quelconque des revendications 1 à 6, **caractérisé en ce que** l'ensemble (1) présente un dispositif de caméra (6), dans lequel le dispositif de caméra (6) de préférence
- présente un capteur d'inclinaison, de préférence un capteur d'accélération, et/ou
- est en liaison de données avec le premier capteur (2) et/ou le deuxième capteur (3) de préférence sans fil, de manière particulièrement préférée par Bluetooth, WLAN ou ZigBee, et/ou
- est un smartphone.

8. Ensemble selon l'une quelconque des revendications 1 à 7, **caractérisé en ce que** l'ensemble (1) présente un dispositif de traitement de données (7), dans lequel le dispositif de traitement de données (7) de préférence :
- est en liaison de données avec le premier capteur (2) et/ou le deuxième capteur (3) de préférence sans fil, de manière particulièrement préférée par Bluetooth, WLAN ou ZigBee, et/ou
- est en liaison de données avec le dispositif de caméra (6) de préférence sans fil, de manière particulièrement préférée par Bluetooth, WLAN ou ZigBee.

9. Ensemble selon la revendication 8, **caractérisé en ce que** le dispositif de traitement de données (7) est réalisé pour le traitement de données de capteur du premier capteur (2) et/ou du deuxième capteur (3) et/ou pour le traitement de données du dispositif de caméra (6).

10. Ensemble selon l'une quelconque des revendications 8 ou 9, **caractérisé en ce que** le dispositif de traitement de données (7) comprend un articulateur numérique, et/ou est réalisé pour la sortie de données pouvant être utilisées dans un articulateur mécanique.

11. Procédé pour la détection d'une position, en particulier d'un angle de roulis, de tangage, et/ou de lacet, d'un maxillaire supérieur et/ou d'une tête humain(e) avec un ensemble selon l'une quelconque des revendications 1 à 10, **caractérisé par** les étapes suivantes :
- la disposition d'un premier capteur (2) sur un maxillaire supérieur ou une tête,
- la détection d'une position du maxillaire supérieur et/ou de la tête par l'intermédiaire du premier capteur (2),
- la transmission de la position détectée du maxillaire supérieur et/ou de la tête par l'intermédiaire de l'interface de données (2c),
- de préférence dans lequel le premier capteur (2) est étalonné avant la première étape de procédé.

12. Procédé pour la détection d'une position, en particulier d'un angle de roulis, de tangage, et/ou de lacet, d'un maxillaire supérieur et/ou d'une tête humain(e) avec un ensemble selon l'une quelconque des revendications 1 à 10, **caractérisé par** les étapes suivantes :
- la disposition d'un premier capteur (2) sur un maxillaire supérieur,
- la disposition d'un deuxième capteur (3) sur une tête,
- la synchronisation du deuxième capteur (3) avec le premier capteur (2),
- le retrait du premier capteur (2), et
- la détection d'une position du maxillaire supérieur et/ou de la tête par l'intermédiaire du deuxième capteur (3),
- de préférence dans lequel le premier capteur (2) est étalonné avant la première étape de procédé.

13. Procédé selon l'une quelconque des revendications 11 ou 12, **caractérisé en ce qu'**en tant qu'autre étape au moins une photographie, de préférence au moins respectivement une photographie frontale et une latérale, du maxillaire supérieur et/ou de la tête est prise par l'intermédiaire d'un dispositif de caméra (6), de préférence dans lequel
- lors de la prise de l'au moins une photographie une position, en particulier un angle de roulis, de tangage, et/ou de lacet, du dispositif de caméra (6) est détectée par l'intermédiaire d'un capteur d'inclinaison, de préférence d'un capteur d'accélération, du dispositif de caméra (6), et/ou
- l'au moins une photographie et/ou une position de l'au moins une photographie est transmise à partir du dispositif de caméra (6) sur un dispositif de traitement de données (7).

14. Procédé selon l'une quelconque des revendications 11 à 13, **caractérisé en ce que** la position détectée du maxillaire supérieur et/ou de la tête est transmise à partir du premier capteur (2) ou du deuxième capteur (3) sur un dispositif de traitement de données (7), de préférence dans lequel un modèle d'un maxillaire supérieur et/ou d'une tête est positionné dans la bonne position par l'intermédiaire de la position détectée du maxillaire supérieur et/ou de la tête.

15. Procédé selon les revendications 13 et 14, **caractérisé en ce que** dans le dispositif de traitement de données (7) l'au moins une photographie est positionnée dans la bonne position par rapport au modèle du maxillaire supérieur et/ou de la tête par l'intermédiaire de la position détectée de la photographie, de préférence dans lequel l'au moins une photographie est mise à l'échelle en fonction du modèle du maxillaire supérieur et/ou de la tête.
